## Europäisches Patentamt

## European Patent Office

## Office européen des brevets

(11) Veröffentlichungsnummer: **0 125 689**
**B2**

(12) **NEUE EUROPÄISCHE PATENTSCHRIFT**

(45) Veröffentlichungstag der neuen Patentschrift:
**18.10.89**

(21) Anmeldenummer: **84105513.0**

(22) Anmeldetag: **16.05.84**

(51) Int. Cl.⁴: **B 01 J 23/80, C 07 C 29/15**

(54) Katalysator für die Methanolsynthese.

(30) Priorität: **16.05.83 DE 3317725**

㊸ Veröffentlichungstag der Anmeldung
**21.11.84 Patentblatt 84/47**

(45) Bekanntmachung des Hinweises auf die Patenterteilung:
**16.06.87 Patentblatt 87/25**

(45) Bekanntmachung des Hinweises auf die Entscheidung
über den Einspruch:
**18.10.89 Patentblatt 89/42**

(84) Benannte Vertragsstaaten:
**BE DE FR GB NL**

(56) Entgegenhaltungen:
**EP-A- 0 042 471**
**FR-A- 2 037 567**
**US-A- 3 388 972**
**US-A- 3 407 030**
**US-A- 3 661 806**

**"Preparation of Catalysts III"', Vorabdruck eines
Vortrages von Gherardi et al, gehalten auf der
Katalysatortagung in Leuwen, 1982**

(73) Patentinhaber: **SÜD-CHEMIE AG, Lenbachplatz 6,
D-8000 München 2 (DE)**

(72) Erfinder: **Schneider, Michael, Dr. Dipl.-Chem.,
Waldparkstrasse 54a, D-8012 Ottobrunn-Riemerling
(DE)**
Erfinder: **Kochloefl, Karl, Dr. Dipl.-Chem.,
Kreuzstrasse 2, D-8052 Moosburg (DE)**
Erfinder: **Ladebeck, Jürgen, Dr. Dipl.-Chem.,
Konradweg 10, D-8300 Landshut (DE)**

(74) Vertreter: **Reitzner, Bruno, Dr. et al, Patentanwälte
Dipl.-Ing. R. Splanemann Dr. B. Reitzner Tal 13,
D-8000 München 2 (DE)**

## Beschreibung

Die Erfindung betrifft einen Katalysator für die Methanolsynthese, enthaltend (a) als katalytisch wirksame Substanzen Kupferoxid und Zinkoxid, wobei gegebenenfalls wenigstens das Kupferoxid mindestens teilweise reduziert ist, und (b) als thermostabilisierende Substanz Aluminiumoxid.

Derartige Katalysatoren, welche die Umsetzung von CO, $CO_2$ und $H_2$ zu Methanol katalysieren, sind schon seit längerer Zeit bekannt. Sie ergeben bei Temperaturen von 200 bis 300°C und Drucken zwischen 30 und 250 bar befriedigende Ausbeuten an Methanol und weisen gute Standseiten auf. Die Atomverhältnisse zwischen Kupfer und Zink können bei diesen bekannten Katalysatoren variieren, wobei jedoch das Kupfer im allgemeinen im Überschuss vorliegt. Ferner kann ein Teil der Zinkkomponente teilweise durch Calcium, Magnesium und/oder Mangan ersetzt sein. Das als thermostabilisierende Substanz verwendete Aluminiumoxid kann teilweise auch durch Chromoxid ersetzt sein. Derartige Katalysatoren sind beispielsweise aus den deutschen Offenlegungsschriften 1 956 007, 2 302 658 und 2 056 612 sowie aus der US-PS 4 279 781 bekannt.

Insbesondere lehrt die US-PS 4 279 781, dass durch die Verwendung von schwer reduzierbaren Metalloxiden, wie Aluminiumoxid, die Thermoresistenz des Cu/Zn-Systems verbessert werden kann, wobei deutlich längere Standzeiten erhalten werden. Der Zusatz an thermostabilisierenden Substanzen muss sich jedoch in Grenzen halten, da bei einem zu hohen Anteil dieser Substanzen die Selektivität und die Aktivität der Cu/Zn-Katalysatoren beeinträchtigt werden.

Der Erfindung liegt die Aufgabe zugrunde, Katalysatoren der eingangs definierten Gattung bereitzustellen, die bei guter Aktivität und Selektivität eine hohe Thermoresistenz oder thermische Stabilität aufweisen. Bei einer hohen Thermoresistenz verlängert sich die Standzeit der Katalysatoren, bzw. sie können bei höheren Temperaturen eingesetzt werden, bei denen die Abwärme der Synthesereaktion wirtschaftlich besser genutzt werden kann.

Es wurde überraschenderweise gefunden, dass diese Aufgabe bei den eingangs definierten Katalysatoren durch eine bestimmte Porenstruktur gelöst werden kann, derart, dass der Anteil der Poren mit einem Durchmesser im Bereich von 20 bis 75 Å (die nachstehend als Mesoporen bezeichnet werden), mindestens 20% und der Anteil der Poren mit einem Durchmesser von mehr als 75 Å (die nachstehend als Makroporen bezeichnet werden), höchstens 80% beträgt.

Vorzugsweise liegt der Anteil der Mesoporen im Bereich von 20 bis 40, insbesondere im Bereich von 30 bis 40%, und der Anteil der Makroporen im Bereich von 80 bis 60, insbesondere von 70 bis 60%. Der Anteil der Poren mit einem Porendurchmesser von weniger als 20 Å (Mikroporen) ist verhältnismässig gering und liegt im allgemeinen unter 0,5%.

Aufgrund der erfindungsgemässen Porenverteilung ist es möglich, den Anteil des als thermostabilisierende Substanz verwendeten Aluminiumoxids zu erhöhen, ohne dass der Umsatz und die Selektivität der Methanolsynthesereaktion beeinträchtigt werden. Dies beruht wahrscheinlich darauf, dass die Synthesereaktion unter praxisnahen Bedingungen innerhalb der Poren des Katalysators stattfindet, wobei die innere Diffusion ein wichtiger Faktor ist und die aktive Oberfläche des Katalysators nicht vollständig ausgenutzt wird. Durch Aktivitätsuntersuchungen an Katalysator-Presslingen verschiedener Grösse konnte festgestellt werden, dass mit abnehmender Grösse der Presslinge der Ausnutzungsgrad sowie die Ausbeute an Methanol ansteigen, weil offenbar die Diffusion der Reaktanten begünstigt und die Oberflächenreaktion geschwindigkeitsbestimmend wird. Gleichzeitig nimmt die Menge an gebildeten organischen Nebenprodukten ab, d.h. die Selektivität verbessert sich, was wahrscheinlich durch die verkürzte Verweilzeit der Reaktionsprodukte bzw. der Zwischenprodukte in den Mesoporen des Katalysators bedingt ist.

Es wurde ferner festgestellt, dass eine optimale Ausbeute an Methanol dann erreicht wird, wenn das Atomverhältnis Cu/Zn zwischen 2,8 und 3,8, vorzugsweise zwischen 2,8 und 3,2, liegt und der Anteil an $Al_2O_3$ 8 bis 12 Gew.-%, vorzugsweise 9 bis 11 Gew.-% beträgt. Unter diesen Bedingungen beträgt der erreichte Ausnutzungsgrad der erfindungsgemässen Katalysatoren (bezogen auf CO) 83 bis 90%.

Die erfindungsgemässen Katalysatoren haben ferner vorzugsweise eine spezifische Oberfläche von mehr als 80 m²/g, vorzugsweise von mehr als 100 m²/g.

Die erfindungsgemässe Porenverteilung kann auf einfache Weise durch Modifizierung der Aluminiumoxidkomponente eingestellt werden. Diese Tatsache ist insofern überraschend, als der Anteil der Aluminiumoxidkomponente im allgemeinen niedriger ist als der Anteil der Kupferoxid-Zinkoxid-Komponente.

Die Aluminiumoxidkomponente kann in dem gewünschten Sinn modifiziert werden, indem bei der Herstellung des Katalysators kolloidal verteiltes Aluminiumoxid oder -hydroxid verwendet wird.

Diese kolloidalen Modifikationen der Aluminiumoxid-Komponente haben im allgemeinen Primärteilchengrössen im Bereich von etwa 1000 bis 10 000 Å.

Sie können in Form von Xerogelen, Hydrogelen oder Solen vorliegen. Xerogele können als trockene Pulver der trockenen Kupferoxid-Zinkoxid-Komponente bzw. einer Vorstufe davon zugemischt werden, worauf das erhaltene Gemisch calciniert wird.

Besonders bevorzugt wird die Aluminiumoxidkomponente jedoch in Form von Hydrogelen und Solen verwendet. Diese können der trockenen Kupferoxid-Zinkoxid-Komponente bzw. deren Vorstufen zugesetzt werden.

Die erfindungsgemässen Katalysatoren werden dadurch erhalten, dass die katalytisch wirksame Kupferoxid-Zinkoxid-Komponente aus wässrigen Lösungen der entsprechenden Salze (z.B. den Nitraten, Sulfaten, Chloriden, Acetaten usw.) mit alkalisch reagierenden Substanzen in Gegenwart des Kolloidal (als Gel oder Sol) verteilten Aluminiumoxids oder -hydroxids ausgefällt wird. Das erhaltene Gemisch bzw. Fällungsprodukt kann in an sich bekannter Weise getrocknet, calciniert, zu Formkörpern verpresst und gegebenenfalls reduziert werden. Die Reduktion des Katalysators kann auch zu Beginn der Methanolsynthesereaktion durchgeführt werden, da das Synthesegas Wasserstoff enthält.

Vorzugsweise verwendet man als alkalisch reagierende Substanzen für die Fällung ein Alkali- oder Ammoniumcarbonat oder -bicarbonat, vorzugsweise Natriumcarbonat oder Natriumbicarbonat. Die Verwendung von Kaliumcarbonat bzw. Kaliumbicarbonat ist weniger zweckmässig, da ein Restgehalt an Kalium im Katalysator die Selektivität der Methanolsynthese verschlechtert, d.h. es bilden sich auch höhere Alkohole. Ein Restgehalt an Natrium im Katalysator stört dagegen nicht, so dass ein mit Natriumcarbonat bzw. Natriumbicarbonat ausgefällter Niederschlag nicht besonders gewaschen zu werden braucht.

Eine weitere Verbesserung der Porenstruktur in Richtung auf die Erhöhung des Anteils der Mesoporen kann dadurch erreicht werden, dass die Fällung der Kupferoxid-Zinkoxid-Komponente in Gegenwart des kolloidal verteilten Aluminiumoxids oder -hydroxids unter Verwendung von verhältnismässig verdünnten Lösungen der alkalisch reagierenden Substanzen, bei verhältnismässig niedrigen Temperaturen und bei neutralen bzw. sogar schwach sauren pH-Werten durchgeführt wird.

Vorzugsweise wird die Fällung mit einer $NaHCO_3$- bzw. $Na_2CO_3$-Lösung mit einer Konzentration von 5 bis 17 Gew.-%, vorzugsweise von 8 bis 12 Gew.-% durchgeführt. Die Fällungstemperatur beträgt zweckmässig 25 bis 65°C, vorzugsweise 30 bis 40°C. Der Zusatz der alkalisch reagierenden Substanzen wird im allgemeinen beendet, wenn der pH-Wert im Bereich von 6,5 bis 7,5, vorzugsweise von 6,9 bis 7,5 liegt.

Bei bekannten Katalysatoren erfolgt die Fällung üblicherweise bei höheren Temperaturen und höheren Lösungskonzentrationen der alkalisch reagierenden Substanzen.

Eine weitere bevorzugte Massnahme zur Verbesserung der gewünschten Porenverteilung besteht darin, dass man das Fällungsprodukt unmittelbar nach seiner Herstellung trocknet, d.h. eine Kristallreifung und Alterung des Niederschlags in Berührung mit der wässrigen Phase verhindert.

Die Erfindung ist durch die nachstehenden Beispiele erläutert:

Vergleichsbeispiel

Zur Fällung des Katalysatorvorläufers werden zwei Lösungen hergestellt:

Lösung 1: 418 g Kupfernitrat und 50 g Zinkoxid werden in 1 Liter Wasser und 148 g 52,5%iger $HNO_3$ gelöst und anschliessend mit einer Lösung von 93,8 g Al $(NO_3)_3 \cdot 9H_2O$ in 0,5 Liter $H_2O$ versetzt.

Lösung 2: 410 g Natriumcarbonat werden in 2 Liter gelöst.

Die Lösungen werden getrennt auf 68°C erwärmt und unter starkem Rühren in der Weise vereinigt, dass der pH-Wert während der Fällung 6,7 beträgt. Der Niederschlag wird unter Rühren bei 68°C noch eine Stunde in der Mutterlauge gealtert. Anschliessend wird abfiltriert und mit Wasser natriumfrei gewaschen. Der Filterkuchen wird bei 120°C getrocknet und anschliessend 8 Stunden bei 280°C calciniert.

Das calcinierte Produkt wird zerkleinert und nach Zusatz von 2 Gew.-% Graphit verpresst.

Beispiel 1

Es wird analog der im Vergleichsbeispiel angegebenen Arbeitsweise gearbeitet, wobei aber anstelle der Aluminiumnitratlösung ein kolloidales Aluminiummetahydrat-Sol (A10[OH]-Sol) (äquivalente $Al_2O_3$-Menge) verwendet wurde. Diese Suspension wurde bei 50°C unter Rühren langsam mit 30 g der 52,5%igen Salpetersäure versetzt, um die Aluminiummetahydrat-Teilchen zu peptisieren. Anschliessend wurde das Sol mit der Kupfer-Zinknitrat-Lösung vereinigt. Die Fällung und Weiterverarbeitung des Katalysatorvorläufers erfolgte wiederum wie im Vergleichsbeispiel angegeben.

Beispiel 2

Die Arbeitsweise von Beispiel 1 wird mit der Abweichung wiederholt, dass die Aluminiummetahydrat-Suspension und die Metallnitratlösung bei 40°C vereinigt werden.

Beispiel 3

Die Arbeitsweise von Beispiel 1 wird mit der Abweichung wiederholt, dass die Fällung bei einem pH-Wert von 6,9 durchgeführt wird.

Beispiel 4

Zur Fällung des Katalysatorvorläufers werden zwei Lösungen hergestellt:

Lösung 1: 418 g Kupfernitrat und 50 g Zinkoxid werden in 1,6 Liter Wasser und 128 g 52,5%iger $HNO_3$ gelöst und anschliessend mit dem kolloidalen Aluminiummetahydrat-Gel versetzt.

Lösung 2: 410 g Natriumcarbonat werden in 3317 g Wasser gelöst.

Die Fällung und die Weiterverarbeitung des Katalysatorvorläufers erfolgt analog dem Vergleichsbeispiel.

Beispiel 5

Zur Fällung des Katalysatorvorläufers werden zwei Lösungen, wie in Beispiel 4 beschrieben, hergestellt.

Diese werden bei 40°C unter starkem Rühren in der Weise vereinigt, dass der pH-Wert während der Fällung 6,9 beträgt. Nach Abschluss der Fällung, wobei der pH den Wert 7,1 noch nicht überschritten haben soll, wird der Niederschlag abfiltriert und mit Wasser gewaschen. Die Weiterverarbeitung des Katalysatorvorläufers erfolgt analog dem Vergleichsbeispiel.

Die chemische Zusammensetzung der Katalysatorvorläufer ist in Tabelle I angegeben.

Tabelle I

Chemische Zusammensetzung der Katalysatorvorläufer

| Beispiele Nr. | CuO (Gew.-%) | ZnO (Gew.-%) | $Al_2O_3$ (Gew.-%) |
|---|---|---|---|
| Vergleichsbeispiel | 67,7 | 22,2 | 10,1 |
| Beispiel 1 | 65,9 | 22,9 | 11,2 |
| Beispiel 2 | 67,6 | 22,4 | 10,0 |
| Beispiel 3 | 65,0 | 23,1 | 11,9 |
| Beispiel 4 | 67,4 | 21,4 | 11,1 |
| Beispiel 5 | 65,1 | 22,9 | 12,0 |

* Analyse des geglühten, graphitfreien Katalysatorvorläufers

Die physikalischen Eigenschaften der Katalysatorvorläufer sind in Tabelle II angegeben (siehe Tabelle II Seite 5 f.).

Anmerkungen zu Tabelle II:

Die spezifische Oberfläche wurde nach der BET-Methode bestimmt. Das Porenvolumen und der Porendurchmesser wurden wie folgt bestimmt:

Das Volumen bzw. die Porengrössenverteilung für Poren bis hinunter zu 75 Å wurde mit Hilfe der Quecksilberporosimetrie ermittelt. In einer zweiten Messung wurde unter Anwendung der $N_2$-Porosimetrie (BET-Methode) das Porenvolumen sowie die Verteilung für Poren unterhalb 1000 Å bestimmt; der Anteil des Porenvolumens, welcher auf die Poren < 75 Å entfiel, wurde berechnet und dem mit Hilfe der Hg-Porosimetrie ermittelten Volumen hinzugefügt.

Die Aktivität der erfindungsgemässen Katalysatoren bei der Methanolsynthese wurde wie folgt getestet.

30 ml Katalysatorvorläufer in Form von Presslingen wurden zunächst drucklos im Reaktionsrohr reduziert, wobei die Presslinge in strömendem (50 Liter/h) Reduktionsgas ($1,2\%$ $H_2$, Rest $N_2$) temperaturprogrammiert auf 235°C aufgeheizt wurden. Von 235°C bis zum Erreichen der Synthesetemperatur (250°C) wurde mit reinem $H_2$ gespült.

Dann wurde ein Synthesegas mit folgender Zusammensetzung (in Vol.-%) über die Katalysatorpresslinge geleitet: $H_2 = 75,3\%$, $CO = 10,8\%$, $CO_2 = 3,9\%$; Inertgase = 10% ($CH_4 = 7,2\%$, $N_2 = 2,8\%$).

Die Reaktionsbedingungen waren wie folgt:

Eingangstemperatur 250°C

Druck 50 bar

Tabelle II

| Beispiele | spez. Oberfläche $(m^2/g)$ | spez. Gewicht (g/liter) | Porenvolumen (ml/g) | Porendurchmesser | |
|---|---|---|---|---|---|
| | | | | 75–20 Å % | 75 Å (%) |
| Vergleichsbeispiel | 63 | 1280 | 0,24 | 18 | 82 |
| Beispiel 1 | 113 | 1060 | 0,36 | 39 | 61 |
| Beispiel 2 | 113 | 1040 | 0,42 | 34 | 66 |
| Beispiel 3 | 107 | 1040 | 0,34 | 32 | 68 |
| Beispiel 4 | 100 | 1070 | 0,34 | 42 | 58 |
| Beispiel 5 | 127 | 1025 | 0,44 | 43 | 57 |

Belastung (HSV) = 10000 Vol. Synthesegas/ Vol. Katalysator und Stunde.

Die Ergebnisse der Aktivitätsmessungen sind in Tabelle III angegeben. Die Methanolausbeute ist bei den erfindungsgemässen Katalysatoren höher als bei dem Vergleichskatalysator. Zu bemerken ist ferner, dass die Methanolausbeute als Folge des höheren Ausnutzungsgrades bei solchen Katalysatoren besonders erhöht ist, deren Vorläufer bei Temperaturen unterhalb 60°C, bei einem leicht erhöhten pH-Wert und aus verdünnteren Lösungen als üblich ausgefällt wurden.

## Tabelle III

| Beispiel | Raum-Zeit-Ausbeute an Reinmethanol (kg/l · h) | Ausnutzungsgrad $\eta_{CO}$ (%) | Organische Nebenprodukte* (Gew.-%) |
|---|---|---|---|
| Vergleichsbeispiel | 0.994 | 80.9 | 0.3 |
| Beispiel 1 | 1.090 | 87.9 | 0.4 |
| Beispiel 2 | 1.076 | 85.7 | 0.4 |
| Beispiel 3 | 1.029 | 83.4 | 0.4 |
| Beispiel 4 | 1.088 | 88.3 | 0.3 |
| Beispiel 5 | 1.103 | 90.2 | 0.4 |

\* Der Hauptanteil besteht aus aliphatischen Alkoholen ($C_2$-$C_5$) mit einem Maximum bei $C_2H_5OH$.

## Patentansprüche

1. Katalysator für die Methanolsynthese, enthaltend

a) als katalytisch wirksame Substanzen Kupferoxid und Zinkoxid, wobei gegebenenfalls wenigstens das Kupferoxid mindestens teilweise reduziert ist, und

b) als thermostabilisierende Substanz Aluminiumoxid, dadurch gekennzeichnet, dass der Anteil der Poren mit einem Durchmesser im Bereich von 20 bis 75 Å mindestens 20% und der Anteil der Poren mit einem Durchmesser von mehr als 75 Å höchstens 80% beträgt.

2. Katalysator nach Anspruch 1, dadurch gekennzeichnet, dass der Anteil der Poren mit einem Durchmesser im Bereich von 20 bis 75 Å im Bereich von 20 bis 40% und der Anteil der Poren mit einem Durchmesser von mehr als 75 Å im Bereich von 80 bis 60% liegt.

3. Katalysator nach Anspruch 1 oder 2, dadurch gekennzeichnet, dass das Atomverhältnis Cu/Zn zwischen 2,8 und 3,8, vorzugsweise zwischen 2,8 und 3,2 liegt und der Anteil an $Al_2O_3$ 8 bis 12 Gew.-% beträgt.

4. Katalysator nach einem der Ansprüche 1 bis 3, dadurch gekennzeichnet, dass er im nicht reduzierten Zustand eine spezifische Oberfläche von mehr als 80 m²/g, vorzugsweise mehr als 100 m²/g besitzt.

5. Katalysator nach einem der Ansprüche 1 bis 4, dadurch erhältlich, dass zu seiner Herstellung kolloidal verteiltes Aluminiumoxid oder -hydroxid verwendet wird.

6. Katalysator nach einem der Ansprüche 1 bis 5, dadurch erhältlich, dass die katalytisch wirksame Kupferoxid-Zinkoxid-Komponente aus wässrigen Lösungen der entsprechenden Salze mit alkalisch reagierenden Substanzen in Gegenwart von kolloidal verteiltem Aluminiumoxid oder -hydroxid ausgefällt und das erhaltene Gemisch in an sich bekannter Weise getrocknet, calciniert, zu Formkörpern verpresst und gegebenenfalls reduziert wird.

7. Katalysator nach einem der Ansprüche 1 bis 6, dadurch erhältlich, dass die Fällung mit einem Alkali- oder Ammoniumcarbonat ober -bicarbonat, vorzugsweise mit Natriumcarbonat oder Natriumbicarbonat, durchgeführt wird.

8. Katalysator nach einem der Ansprüche 1 bis 7, dadurch erhältlich, dass die Fällung mit einer $NaHCO_3$- bzw. $Na_2CO_3$-Lösung mit einer Konzentration von 5 bis 17, vorzugsweise von 8 bis 11 Gew.-%, durchgeführt wird.

9. Katalysator nach einem der Ansprüche 1 bis 8, dadurch erhältlich, dass die Fällung bei Temperaturen von 25 bis 65°C, vorzugsweise von 30 bis 40°C, durchgeführt wird.

10. Katalysator nach einem der Ansprüche 1 bis 9, dadurch erhältlich, dass die Fällung bei pH-Werten im Bereich von 6,5 bis 7,5, vorzugsweise von 6,9 bis 7,5 durchgeführt wird.

11. Katalysator nach einem der Ansprüche 1 bis 10, dadurch erhältlich, dass das Fällungsprodukt unmittelbar nach seiner Herstellung von der Mutterlauge abgetrennt, gewaschen und getrocknet wird.

## Claims

1. A catalyst for methanol synthesis, containing

a) copper oxide and zinc oxide as catalytically active substances, wherein optionally at least the copper oxide is at least partly reduced, and

b) aluminium oxide as a thermostabilising substance, characterised in that the proportion of pores with a diameter within a range of from 20 to 25 Å is at least 20% and the proportion of pores with a diameter of more than 75 Å is at maximum 80%.

2. A catalyst according to Claim 1, characterised in that the proportion of pores with a diameter within a range of from 20 to 75 Å lies within a range of from 20 to 40% and the proportion of pores with a diameter of more than 75 Å lies within a range of from 80 to 60%.

3. A catalyst according to Claim 1 or 2, characterised in that the atomic ratio Cu/Zn is between 2.8 and 3.8, preferably between 2.8 and 3.2 and the proportion of $Al_2O_3$ is 8 to 12% by weight.

4. A catalyst according to any of Claims 1 to 3, characterised in that in the unreduced state it has a specific surface of more than 80 m²/g, preferably more than 100 m²/g.

5. A catalyst according to any of Claims 1 to 4, which can be obtained by using colloidally dispersed aluminium oxide or hydroxide for its preparation.

6. A catalyst according to any of Claims 1 to 5, which can be obtained by precipitating the catalytically active cooper oxide-zinc oxide component from aqueous solutions of the corresponding salts with alkaline reagents, in the presence of colloidally dispersed aluminium oxide or hydroxide, and in per se known manner the resultant mixture is dried, calcined, pressed to form moulded bodies and, optionally, reduced.

7. A catalyst according to any of Claims 1 to 6, which can be obtained by carrying out the precipitation with an alkali or ammonium carbonate or bicarbonate, preferably with sodium carbonate or sodium bicarbonate.

8. A catalyst according to any of Claims 1 to 7, which can be obtained by carrying out the precipitation with a solution of $NaHCO_3$ or $Na_2CO_3$ at a concentration of from 5 to 17, preferably from 8 to 11% by weight.

9. A catalyst according to any of Claims 1 to 8, which can be obtained by carrying out the precipitation at temperatures of from 25 to 65°C, preferably from 30 to 40°C.

10. A catalyst according to any of Claims 1 to 9, which can be obtained by carring out the precipitation at pH values within a range of from 6.5 to 7.5, preferably from 6.9 to 7.5.

11. A catalyst according to any of Claims 1 to 10, which can be obtained in that, immediately after its preparation, the precipitation product is separated from the mother liquor, washed and then dried.

Revendications

1. Catalyseur pour la synthèse de méthanol, contenant

a) comme substance active catalytiquement, de l'oxyde de cuivre et de l'oxyde de zinc, au moins l'oxyde de cuivre étant éventuellement réduit au moins partiellement, et

b) comme substance thermostabilisante, de l'oxyde d'aluminium, caractérisé par le fait que la proportion des pores ayant un diamètre de la gamme de 20 à 75 Å est d'au moins 20% et que la proportion des pores ayant un diamètre de plus de 75 Å est au maximum de 80%.

2. Catalyseur selon la revendication 1, caractérisé par le fait que la proportion des pores ayant un diamètre de la gamme de 20 à 75 Å se situe dans la gamme de 20 à 40% et que la proportion des pores ayant un diamètre de plus de 75 Å se situe dans la gamme de 80 à 60%.

3. Catalyseur selon l'une des revendications 1 et 2, caractérisé par le fait que le rapport atomique Cu/Zn se situe entre 2,8 et 3,8, de préférence entre 2,8 et 3,2, et que la proportion d'$Al_2O_3$ est de 8 à 12% en poids.

4. Catalyseur selon l'une des revendications 1 à 3, caractérisé en ce qu'il présente dans l'état non réduit une surface spécifique de plus de 80 m²/g, de préférence de plus de 100 m²/g.

5. Catalyseur selon l'une des revendications 1 à 4, pouvant être obtenu en utilisant, pour sa préparation, de l'oxyde ou de l'hydroxyde d'aluminium distribué de façon colloïdale.

6. Catalyseur selon l'une des revendications 1 à 5, pouvant être obtenu en précipitant le constituant oxyde de cuivre-oxyde de zinc, actif catalytiquement, de solutions aqueuses des sels correspondants, avec des substances à réaction alcaline, en présence d'oxyde ou hydroxyde d'aluminium distribué de façon colloïdale, et de manière en elle-même connue, en séchant le mélange obtenu, en le calcinant, en le comprimant en pièces moulées et éventuellement en le réduisant.

7. Catalyseur selon l'une des revendications 1 à 6, pouvant être obtenu en conduisant la précipitation avec un carbonate ou bicarbonate alcalin ou d'ammonium, de préférence avec du carbonate de sodium ou du bicarbonate de sodium.

8. Catalyseur selon l'une des revendications 1 à 7, pouvant être obtenu en effectuant la précipitation avec une solution de $NaHCO_3$ ou $Na_2CO_3$ d'une concentration de 5 à 17, de préférence de 8 à 11% en poids.

9. Catalyseur selon l'une des revendications 1 à 8, pouvant être obtenu en effectuant la précipitation à des températures de 25 à 65°C, de préférence de 30 à 40°C.

10. Catalyseur selon l'une des revendications 1 à 9, pouvant être obtenu en effectuant la précipitation à des pH de la gamme de 6,5 à 7,5, de préférence de 6,9 à 7,5.

11. Catalyseur selon l'une des revendications 1 à 10, pouvant être obtenu en séparant le produit de précipitation de la liqueur mère immédiatement après sa préparation, qu'on le lave et qu'on le sèche.